# EUROPEAN PATENT APPLICATION

(11) **EP 3 338 767 A1**
(43) Date of publication of application: **27.06.2018**
(21) Application number: 17209510.1
(22) Date of filing: 21.12.2017
(51) Int. Cl.: A61K 9/48, A61K 9/20, A61K 31/13, A61K 31/445, A61K 9/50

(54) **CAPSULE COMPOSITIONS COMPRISING DONEPEZIL AND MEMANTINE**

(30) Priority: 22.12.2016 TR 201619184
(71) Applicant: Sanovel Ilac Sanayi ve Ticaret A.S., 34460 Istanbul (TR)
(72) Inventor: TÜRKYILMAZ, Ali, 34460 Istanbul (TR); YILDIRIM, Ediz, 34460 Istanbul (TR); MURATOGLU, Yesim, 34460 Istanbul (TR)
(74) Representative: Sevinç, Erkan

(57) **Abstract**

The present invention relates to a capsule dosage form comprising donepezil or a pharmaceutically acceptable salt thereof in combination with memantine or a pharmaceutically acceptable salt thereof.

## Description

### Field of Invention

The present invention relates to a capsule dosage form comprising donepezil or a pharmaceutically acceptable salt thereof in combination with memantine or a pharmaceutically acceptable salt thereof.

### The background of the invention

Acetylcholinesterase inhibitors (often abbreviated AChEI) are drugs that inhibit the acetylcholinesterase enzyme from breaking down acetylcholine, thereby increasing both the level and duration of action of the neurotransmitter acetylcholine. Acetylcholinesterase inhibitors are classified as reversible, irreversible, or pseudo-irreversible.

Donepezil acts as a centrally acting reversible acetylcholinesterase inhibitor. It binds and inactivates reversibly the cholinesterases, thus inhibiting hydrolysis of acetylcholine. This results in increased acetylcholine concentrations at cholinergic synapses.

Donepezil, marketed under the trade name Aricept®, is a medication used in the palliative treatment of Alzheimer's disease. It is used to improve cognition and behavior of people with Alzheimer's, but does not slow the progression of or cure the disease.

Its chemical name is (RS)-2-[(1-Benzyl-4-piperidyl)methyl]- 5,6-dimethoxy-2,3-dihydroinden-1-one and its chemical structure is shown in the Formula 1.

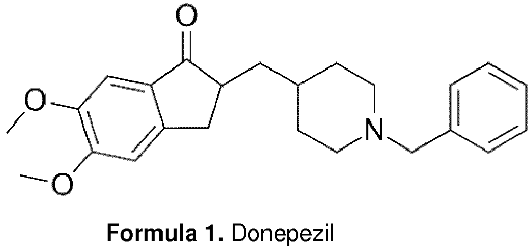

The N-methyl-D-aspartate receptor (also known as the NMDA receptor or NMDAR), is a glutamate receptor and ion channel protein found in nerve cells. It is activated when glutamate and glycine (or D-serine) bind to it, and when activated it allows positively charged ions to flow through the cell membrane. The NMDA receptor is very important for controlling synaptic plasticity and memory function.

Memantine is a NMDAR antagonist. NMDA receptor antagonists are a class of anesthetics that work to antagonize, or inhibit the action of, the NMDAR. Memantine is the first in a novel class of Alzheimer's disease medications acting on the glutamatergic system by blocking NMDA receptors.

Memantine has been shown to have a modest effect in moderate-to-severe Alzheimer's disease and in dementia with Lewy bodies. It is marketed under the brand Namenda® among others, its chemical name is 3,5-dimethyladamantan-1-amine and its chemical structure is shown in the Formula 2.

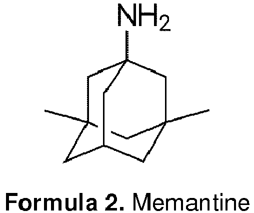

The combination of donepezil and memantine, which is used to treat moderate to severe dementia of the Alzheimer's type, is in the market under the trade name Namzaric®.

In the state of art, patent document numbered EP1509232B1 discloses the use of a composition comprising acetylcholinesterase inhibitor(s) and NMDA-antagonist(s) to treat mild cognitive impairment or dementia in connection with moderate to severe Alzheimer's. The use of donepezil and the use of memantine as active agents are also stated in this patent document.

Another prior art, patent application numbered WO2011127235A1, mentions an Alzheimer's disease treating method comprising the administration donepezil and memantin to the patient.

It is well known that drugs even used in the same therapeutic area can not always be combined in a dosage form due to incompatibility problems. The scientific literature is full of unsuccessful examples wherein compounds, which are used to treat the same indications but having different pharmacokinetic properties, are tried to be combined.

In order to combine two or more different active agents in one dosage form, active agents should be compatible or they should be adjusted to be compatible with each other to achieve desired stability and dissolution profile.

The combination of donepezil and memantin and its medical indications are already known. However, there are very few attempts to formulate stable and efficient formulations comprising them. The main challenge about this combination is the difference between these active agents' aqueus solubilities and dissolution rates. The solubility of memantin is much higher than the solubility of donepezil. These difference brings many undesirable consequences, especially in bioavailability, relating the inconsistent, inefficient and nonhomogeneous distribution of them. This situation also restrains the synergic effect of these two active agents in the combination.

In the state of art, a triple combination comprising donepezil, memantine and ginkgo biloba extract is mentioned. The technical problem to be solved by this combination is the nonhomogeneous distribution of donepezil due to the amount of ginkgo biloba extract in the formulation which is 10 times more than the amount of donepezil. The approach to this problem draws attention to the selection of the binder; therefore, it is stated that sorbitol is the most preferable binder to ensure the bioavailability. It is also emphasized that effervescent form of the composition is preffered to reach the high bioavailability.

Considering the state of art, there is still a need for a dosage form, especially a capsule formulation with mini tablets, comprising a combination of donepezil and memantine which ensures the required dissolution profiles and accordingly safe and efficient plasma levels of both active agents to provide a high patient compliance, bioavailability and stability.

### Objects and Brief Description of the Invention

The main object of the present invention is to obtain combination formulations comprising donepezil and memantine eliminating all aforesaid problems and bringing additional advantages to the relevant prior art.

Another object of the present invention is to obtain combination formulations of donepezil and memantine with high stability, bioavalibility and patient compliance.

A further object of the present invention is to develop combination formulations of donepezil and memantine having an improved level of dissolution rate and solubility.

Another object of the present invention is to provide an immediate release capsule form comprising donepezil and memantine.

A further object of the present invention is to provide a capsule dosage form comprising donepezil and memantine preferably in separate mini tablets.

Yet another object of the present invention is to apply different coatings on donepezil mini tablet(s) and memantine mini tablet(s), which exhibit different solubilities in water, to obtain adequate dissolution profiles from each of them.

### Detailed description of the invention

In accordance with the objects outlined above, detailed features of the present invention are given herein.

The present invention relates to solid oral pharmaceutical compositions comprising donepezil or a pharmaceutically acceptable salt thereof in combination with memantine or a pharmaceutically acceptable salt thereof as active agents and at least one pharmaceutically acceptable excipient.

According to the preferred embodiment of the invention, the active agents are donepezil hydrochloride (donepezil HCl) and memantine hydrochloride (memantine HCl).

According to one preferred embodiment, the amount of donepezil HCl is between 1-30% by weight of the total composition. Preferably this amount is between 1-20% by weight of the total composition. More preferably donepezil HCl is present between 2-10% by weight in the total composition.

According to this preferred embodiment, the amount of memantine HCl is between 1-40% by weight of the total composition. Preferably this amount is between 3-30% by weight of the total composition. More preferably memantine HCl is present between 5-15% by weight in the total composition.

According to one embodiment, donepezil HCl is present in an amount of 1 to 50 mg, more preferably 5 to 10 mg in the total composition.

Accorrding this embodiment, memantine HCl is present in an amount of 1 to 50 mg, more preferably 5 to 20 mg in the total composition.

In the preferred embodiment of the invention, the ratio of donepezil HCl to memantine HCl is in the range of 1:0.5 to 1:8 and preferably 1:1 to 1:4. The most preferred ratios are 1:1, 1:2, 1:3 and 1:4.

According to these embodiments, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film.

The dosage form of the composition is preferably an immediate release capsule. According the preffered embodiment, the capsule is made of hydroxypropyl methylcellulose (HPMC) or gelatin.

In the capsule, donepezil HCl is present in a dosage form such as tablet, coated tablet, mini tablet, coated mini tablet, coated bead system, powder, pellet or a mixture thereof.

In the capsule, memantine HCl is present in a dosage form such as tablet, coated tablet, mini tablet, coated mini tablet, coated bead system or a mixture thereof.

According to one embodiment, the capsule dosage form subjected to the invention is filled with at least one dosage form of donepezil HCl mini and at least one dosage form of memantine HCl.

According to the preferred embodiment, the capsule dosage form subjected to the invention is filled with at least one donepezil HCl mini tablet and at least one memantine HCl mini tablet.

The term "mini tablet", as used herein, refers to a small tablet, comprising the active agent, with a diameter equal to or less than 4 mm and a thickness equal to or less than 3 mm. The mini tablets have round shape and smooth surface to ease coating process.

The capsule form filled with mini tablets has many advantages as the simplicity of dosage adjustement, the chance of composing alternative dosage combinations using one or more equivalent mini tablets together, the opportunity to select excipients separately for each mini tablet, to perform different film coatings which are favorable for each mini tablets having different solubility and accordingly to provide the homogeneous distribution of active agents.

According to the most preferred embodiment, donepezil HCl mini tablets or memantine HCl mini tablets or both of them are coated with film layer.

The dosage form of donepezil HCl comprises donepezil HCl and at least one pharmaceutically acceptable excipient selected from diluents, binders, disintegrants, lubricants, or mixtures thereof.

According to the preferred embodiment of the invention, the ratio of donepezil HCl is between 1-30%, preferably 5-20% and more preferably 10% by weight of the dosage form of donepezil HCl.

According to this embodiment, the dosage form of donepezil HCl comprises donepezil HCl in an amount of 1 to 10 mg and more preferably 2,5 mg.

The dosage form of memantine HCl comprises memantine HCl and at least one pharmaceutically acceptable excipient selected from diluents, lubricants, glidants or mixtures thereof. According to the preferred embodiment the dosage form of memantine HCl is free of disintegrant to provide a homogenous dissolution along the dissolution of the dosage form of donepezil HCl, since memantine HCl already has high solubility and rapid disintegration potential.

According to the preferred embodiment of the invention, the ratio of memantine HCl is between 1-40%, preferably 10-30% and more preferably 20% by weight of the dosage form of memantine HCl.

According to this embodiment, the dosage form of memantine HCl comprises memantine HCl in an amount of 1 to 20 mg and more preferably 5 mg.

According to one embodiment of the invention, the dosage form of donepezil HCl comprises at least one diluent which is selected from the group comprising lactose monohydrate, corn starch, lactose, dibasic calcium phosphate, microcrystalline cellulose, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment of the invention, the dosage form of donepezil HCl comprises two diluents which are lactose monohydrate and corn starch.

The amount of lactose monohydrate is between 10-90%, preferably 30-80% and more preferably 50-70% by weight of the dosage form of donepezil HCl.

The amount of corn starch is between 1-50%, preferably 5-30% and more preferably 10-20% by weight of the dosage form of donepezil HCl.

According to one embodiment of the invention, the dosage form of donepezil HCl comprises at least one disintegrant which is selected from the group comprising microcrystalline cellulose, croscarmellose sodium, sodium carbonate, hydroxylpropyl cellulose (HPC), cross-linked polyvinylpyrrolidone (crospovidon), copovidon, polycarbophil, low-substitue poloxamer, alginic acid and alginates, ion-exchange resins, magnesium aluminum silica, sodium dodecyl sulphate, sodium carboxy methyl cellulose, carboxy methyl cellulose calcium, docusate sodium, guar gum, polyacrylin potasium, sodium alginate, sodium glysin carbonate, sodium lauryl sulphate or mixtures thereof.

According to the preferred embodiment of the invention, the dosage form of donepezil HCl comprises one disintegrant which is microcrystalline cellulose.

The amount of microcrystalline cellulose is between 1-30%, preferably 5-15% by weight of the dosage form of donepezil HCl.

According to one embodiment, the dosage form of donepezil HCl comprises at least one binder which is selected from the group comprising copovidone, copolyvidone, polyvinylpyrrolidone (PVP, povidone), povidone K30, carnauba wax, hydroxypropyl methyl cellulose (hypromellose, HPMC), pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, microcrystalline cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to the preferred embodiment of the invention, the dosage form of donepezil HCl comprises one binder which is hydroxypropyl cellulose.

The amount of hydroxypropyl cellulose is between 1-10%, preferably 1-5% by weight of the dosage form of donepezil HCl.

According to the preferred embodiment of the invention, the ratio of the total weight of disintegrant to the total weight of binder is in the range of 1:1 to 10:1, preferably 2:1 to 8:1 and more preferably 4:1 to 6:1, in the dosage form of donepezil HCl. This preferred selection of range assures the desired dissolution and disintegration profiles.

According to one embodiment of the invention, the dosage form of donepezil HCl comprises comprises at least one lubricant which is selected from the group comprising sodium lauryl sulphate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin or mixtures thereof.

According to the preferred embodiment of the invention, the dosage form of donepezil HCl comprises one lubricant which is magnesium stearate.

The amount of magnesium stearate is between 0.1-5%, preferably 0.2-1% by weight of the dosage form of donepezil HCl.

According to the preferred embodiment of the invention, the dosage form of donepezil HCl is donepezil HCl mini tablet or donepezil HCl powder.

According to the most preferred embodiment of the invention, the dosage form of donepezil HCl is donepezil HCl mini tablet.

According to this embodiment, the donepezil HCl mini tablet preferably comprises at least one coating layer to protect the composition against the moisture and maintain the stability. Suitable coating ingredients are selected from the group comprising hydroxypropylmethyl cellulose (hypromellose), lactose monohydrate, hydroxypropyl cellulose, polyvinyl alcohol (PVA), polyethylene glycol (PEG), talc, polyvinyl alcohol-polyethylene glycol copolymers (Kollicoat IR), ethylcellulose dispersions (Surelease), polyvinylprolidone, polyvinylprolidone-vinyl acetate copolymer (PVP-VA) and all kinds of OpadryTM, pigments, dyes, titanium dioxide, iron oxide or polymethylmetacrylate copolymers and mixtures thereof.

According to the preferred embodiment, the donepezil HCl mini tablet has a hydroxypropyl methylcellulose based coating layer (Opadry) comprising lactose monohydrate, hydroxypropylmethyl cellulose (hypromellose), titanium dioxide, polyethylene glycol (PEG) and optionally one ore more pigment.

According to one embodiment of the invention, the dosage form of memantine HCl comprises at least one diluent which is selected from the group comprising lactose monohydrate, corn starch, lactose, dibasic calcium phosphate, microcrystalline cellulose, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment of the invention, the dosage form of memantine HCl comprises two diluents which are microcrystalline cellulose and lactose monohydrate.

The amount of microcrystalline cellulose is between 10-80%, preferably 20-70% and more preferably 35-55% by weight of the dosage form of memantine HCl.

The amount of lactose monohydrate is between 10-60%, preferably 20-50% and more preferably 30-40% by weight of the dosage form of memantine HCl.

According to one embodiment of the invention, the dosage form of memantine HCl comprises at least one lubricant and at least one glidant which are selected from the group comprising sodium lauryl sulphate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin or mixtures thereof.

According to the preferred embodiment of the invention, the dosage form of memantine HCl comprises two lubricants which are magnesium stearate and talc.

The amount of magnesium stearate is between 0.1-5%, preferably 0.5-2% by weight of the dosage form of memantine HCl.

The amount of talc is between 0.1-5%, preferably 0.5-2% by weight of the dosage form of memantine HCl.

According to the preferred embodiment of the invention, the dosage form of memantine HCl comprises one glidant which is colloidal silicon dioxide.

The amount of colloidal silicon dioxide is between 0.05-1%, preferably 0.1-0.5% by weight of the dosage form of memantine HCl.

According to the preferred embodiment of the invention, the ratio of the total weight of lubricant to the total weight of glidant is in the range of 20:1 to 1:1, preferably 15:1 to 5:1 and more preferably 12:1 to 8:1, in the dosage form of memantine HCl. This preferred selection of range assures the desired dissolution and disintegration profiles.

According to the preferred embodiment of the invention, the dosage form of memantine HCl is memantine HCl mini tablet.

According to this embodiment, the memantine HCl mini tablet preferably comprises at least one coating layer providing extended release to adjust the disintegration rate and distribution of memantine HCl and maintain the homogeneous dissolution. Suitable coating ingredients are selected from the group comprising hydroxypropylmethyl cellulose (hypromellose), lactose monohydrate, hydroxypropyl cellulose, polyvinyl alcohol (PVA), polyethylene glycol (PEG), talc, polyvinyl alcohol-polyethylene glycol copolymers (Kollicoat IR), methacrylic acid - ethyl acrylate copolymer (Kollicoat MAE), polysorbate 80, sodium lauryl sulphate, simethicone emulsions, triacetine, ethylcellulose dispersions (Surelease), polyvinylprolidone, polyvinylprolidone-vinyl acetate copolymer (PVP-VA) and all kinds of OpadryTM, pigments, dyes, titanium dioxide, iron oxide or polymethylmetacrylate copolymers and mixtures thereof.

According to the preferred embodiment, the memantine HCl mini tablet has a methacrylic acid based coating layer to protect the gastric mucosa from the memantine HCl which is an aggressive active agent.

The methacrylic acid based coating comprising methacrylic acid - ethyl acrylate copolymer (1:1 by weight), polysorbate 80, sodium lauryl sulphate, simethicone emulsion and triacetine.

In this invention, to overcome the problems mentioned above, capsule forms containing mini tablets have been developed and desired dissolution rates and stability have been achieved.

According to one embodiment, the solid oral pharmaceutical composition is in the form of a capsule comprising two donepezil HCl mini tablets and one memantine HCl mini tablet.

According to another embodiment, the solid oral pharmaceutical composition is in the form of a capsule comprising two donepezil HCl mini tablets and two memantine HCl mini tablets.

According to another preferred embodiment, the solid oral pharmaceutical composition is in the form of a capsule comprising two donepezil HCl mini tablets and three memantine HCl mini tablets.

According to another preferred embodiment, the solid oral pharmaceutical composition is in the form of a capsule comprising two donepezil HCl mini tablets and four memantine HCl mini tablets.

According to another preferred embodiment, the solid oral pharmaceutical composition is in the form of a capsule comprising four donepezil HCl mini tablets and four memantine HCl mini tablets.

In a preferred embodiment of the invention, capsule dosage form, comprising donepezil HCl powder and memantine HCl mini tablet, is comprised of;
- 1 to 30% of donepezil HCl,
- 10 to 90% of lactose monohydrate,
- 1 to 50% of corn starch,
- 1 to 30% of microcrystalline cellulose,
- 1 to 10% of hydroxypropyl cellulose,
- 0.1 to 5% of magnesium stearate by weight of the donepezil HCl powder and;
- 1 to 40% of memantine HCl,
- 10 to 80% of microcrystalline cellulose,
- 10 to 60% of lactose monohydrate,
- 0.1 to 5% of magnesium stearate,
- 0.1 to 5% of talc,
- 0.05 to 1% of colloidal silicone dioxide,
- 1 to 5% of methacrylic acid based coating by weight of the memantine HCl mini tablet.

In the most preferred embodiment of the invention, capsule dosage form, comprising donepezil HCl mini tablet and memantine HCl mini tablet, is comprised of;
- 1 to 30% of donepezil HCl,
- 10 to 90% of lactose monohydrate,
- 1 to 50% of corn starch,
- 1 to 30% of microcrystalline cellulose,
- 1 to 10% of hydroxypropyl cellulose,
- 0.1 to 5% of magnesium stearate,
- optionally 1 to 5% of hydroxypropyl methylcellulose based coating by weight of the donepezil HCl mini tablet and;
- 1 to 40% of memantine HCl,
- 10 to 80% of microcrystalline cellulose,
- 10 to 60% of lactose monohydrate,
- 0.1 to 5% of magnesium stearate,
- 0.1 to 5% of talc,
- 0.05 to 1% of colloidal silicone dioxide,
- 1 to 5% of methacrylic acid based coating by weight of the memantine HCl mini tablet.

According to all these embodiments, the below given formulations can be used in the solid oral pharmaceutical composition subjected to the invention. These examples are not limiting the scope of the present invention and should be considered under the light of the foregoing detailed disclosure.

### Example 1: Formulations of different dosage forms present in the capsule form

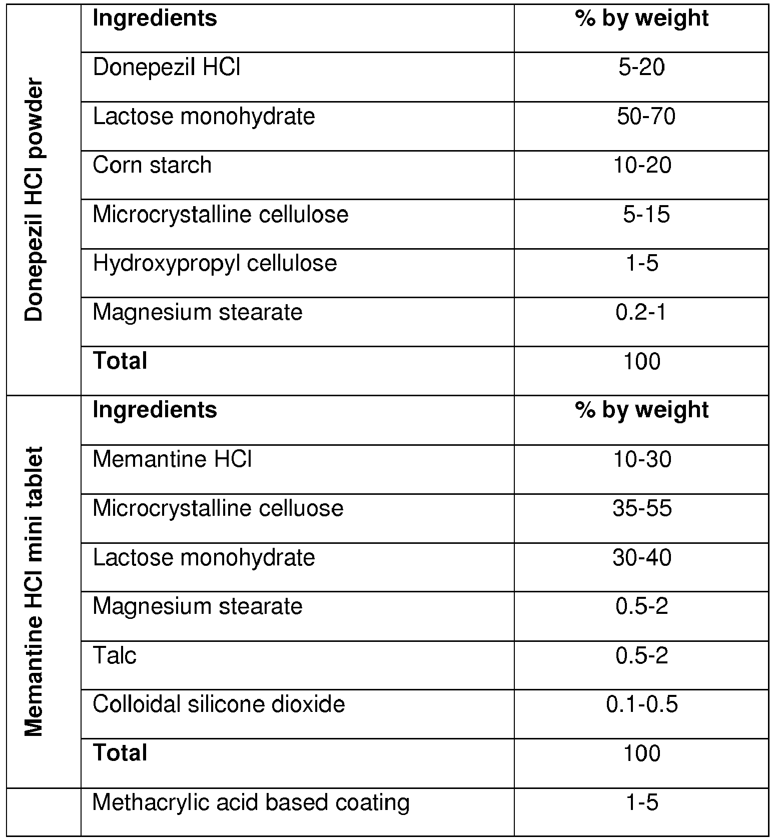

The preparation method of the above mentioned capsule form subjected to the invention are prepared by following these steps:
- Sieving donepezil HCl, lactose monohydrate, corn starch, microcrystalline cellulose and hydroxylpropyl cellulose through a sieving mesh
- Mixing and granulating this sieved mixture with water
- Drying the granules preferably at 50°C in a stove
- Sieving the dried granules
- Adding magnesium stearate and mixing the total donepezil HCl powder mixture
- Sieving memantine HCl, microcrystalline cellulose, lactose monohydrate, colloidal silicone dioxide through a sieving mesh
- Mixing this sieved mixture
- Adding talc and mixing again
- Adding magnesium stearate and mixing again
- Compressing the total mixture to form memantine HCl mini tablets
- Coating the memantine HCl mini tablets with methacrylic acid based coating
- Filling the donepezil HCl powder and the memantine mini tablets into capsules

### Example 2: Formulations of mini tablets present in the capsule form

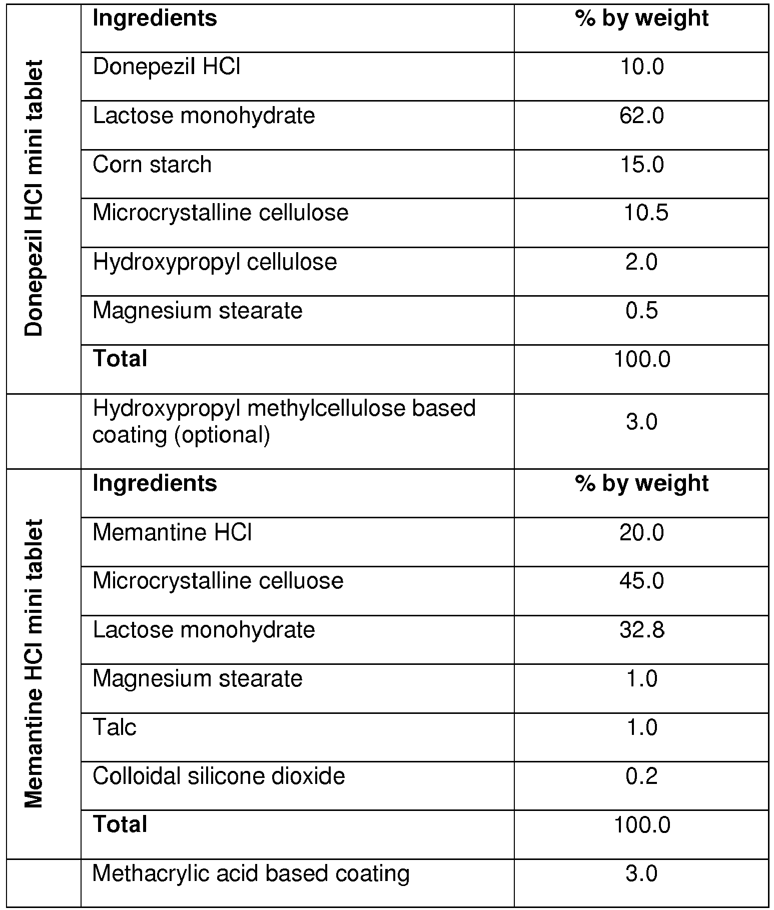

The preparation method of the above mentioned capsule form subjected to the invention are prepared by following these steps:
- Sieving donepezil HCl, lactose monohydrate, corn starch, microcrystalline cellulose and hydroxylpropyl cellulose through a sieving mesh
- Mixing and granulating this sieved mixture with water
- Drying the granules preferably at 50°C in a stove
- Sieving the dried granules
- Adding magnesium stearate and mixing the total mixture
- Compressing the total mixture to form donepezil HCl mini tablets
- Optionally coating the donepezil HCl mini tablets with hydroxypropyl methylcellulose based coating
- Sieving memantine HCl, microcrystalline cellulose, lactose monohydrate, colloidal silicone dioxide through a sieving mesh
- Mixing this sieved mixture
- Adding talc and mixing again
- Adding magnesium stearate and mixing again
- Compressing the total mixture to form memantine HCl mini tablets
- Coating the memantine HCl mini tablets with methacrylic acid based coating
- Filling the donepezil HCl mini tablets and the memantine mini tablets into capsules

## Claims

1. A capsule dosage form comprising donepezil hydrochloride in combination with memantine hydrochloride and at least one pharmaceutically acceptable excipient.

2. The capsule dosage form according to claim 1, wherein the composition comprises donepezil HCl in an amount of 1-30%, preferably 1-20% and more preferably 2-10% by weight of the total composition.

3. The capsule dosage form according to claim 2, wherein the composition comprises memantine HCl in an amount of 1-40%, preferably 3-30% and more preferably 5-15% by weight of the total composition.

4. The capsule dosage form according to claim 3, wherein the ratio of donepezil HCl to memantine HCl is in the range of 1:0.5 to 1:8 and preferably 1:1 to 1:4.

5. The capsule dosage form according to any preceding claims, wherein donepezil HCl and memantine HCl are present in the form of tablet, mini tablet, powder, pellet or a mixture thereof.

6. The capsule dosage form according to claim 5, wherein the capsule is filled with at least one donepezil HCl mini tablet and at least one memantine HCl mini tablet.

7. The capsule dosage form according to claim 6, wherein the donepezil HCl mini tablet comprises donepezil HCl and at least one pharmaceutically acceptable excipient selected from diluents, binders, disintegrants, lubricants, or mixtures thereof.

8. The capsule dosage form according to claim 7, wherein the ratio of donepezil HCl is between 1-30% and preferably 5-20% by weight of the total donepezil HCl mini tablet.

9. The capsule dosage form according to claim 8, wherein the donepezil HCl mini tablet comprises one disintegrant which is microcrystalline cellulose.

10. The capsule dosage form according to claim 9, wherein the donepezil HCl mini tablet comprises one binder which is hydroxypropyl cellulose.

11. The capsule dosage form according to any claim 7 to 10, wherein the ratio of the total weight of disintegrant to the total weight of binder is in the range of 1:1 to 10:1, preferably 2:1 to 8:1 and more preferably 4:1 to 6:1, in the donepezil HCl mini tablet, to assure the desired dissolution and disintegration.

12. The capsule dosage form according to claim 6; wherein the memantine HCl mini tablet, comprising memantine HCl and at least one pharmaceutically acceptable excipient selected from diluents, lubricants, glidants or mixtures thereof, is free of disintegrant to provide the homogenous dissolution.

13. The capsule dosage form according to claim 12, wherein the ratio of memantine HCl is between 1-40% and preferably 10-30% by weight of the total memantine HCl mini tablet.

14. The capsule dosage form according to claim 13, wherein the memantine HCl mini tablet comprises two lubricants which are magnesium stearate and talc, and one glidant which is colloidal silicon dioxide.

15. The capsule dosage form according to claim 14, wherein the ratio of the total weight of lubricant to the total weight of glidant is in the range of 20:1 to 1:1, preferably 15:1 to 5:1 and more preferably 12:1 to 8:1, in the memantine HCl mini tablet, to assure the desired dissolution and disintegration.

16. The capsule dosage form according to any claim 12 to 15, wherein the memantine HCl mini tablet comprises at least one methacrylic acid based coating.

17. The capsule dosage form according to any preceding claims, wherein the composition comprises;
- 1 to 30% of donepezil HCl,
- 10 to 90% of lactose monohydrate,
- 1 to 50% of corn starch,
- 1 to 30% of microcrystalline cellulose,
- 1 to 10% of hydroxypropyl cellulose,
- 0.1 to 5% of magnesium stearate,
- 1 to 5% of hydroxypropyl methylcellulose based coating by weight of the donepezil HCl mini tablet and;
- 1 to 40% of memantine HCl,
- 10 to 80% of microcrystalline cellulose,
- 10 to 60% of lactose monohydrate,
- 0.1 to 5% of magnesium stearate,
- 0.1 to 5% of talc,
- 0.05 to 1% of colloidal silicone dioxide,
- 1 to 5% of methacrylic acid based coating by weight of the memantine HCl mini tablet.

18. A process for preparing the capsule dosage form according to claim 17, comprising the following steps:
- Sieving donepezil HCl, lactose monohydrate, corn starch, microcrystalline cellulose and hydroxylpropyl cellulose through a sieving mesh
- Mixing and granulating this sieved mixture with water
- Drying the granules
- Sieving the dried granules
- Adding magnesium stearate and mixing the total mixture
- Compressing the total mixture to form donepezil HCl mini tablets
- Coating the donepezil HCl mini tablets with hydroxypropyl methylcellulose based coating
- Sieving memantine HCl, microcrystalline cellulose, lactose monohydrate, colloidal silicone dioxide through a sieving mesh
- Mixing this sieved mixture
- Adding talc and mixing again
- Adding magnesium stearate and mixing again
- Compressing the total mixture to form memantine HCl mini tablets
- Coating the memantine HCl mini tablets with methacrylic acid based coating
- Filling the donepezil HCl mini tablets and the memantine mini tablets into capsules
